(19) **Europäisches Patentamt**
**European Patent Office**
**Office européen des brevets**

(11) **EP 2 424 579 B1**

(12) **FASCICULE DE BREVET EUROPEEN**

(45) Date de publication et mention
de la délivrance du brevet:
**02.09.2015 Bulletin 2015/36**

(21) Numéro de dépôt: **10715551.7**

(22) Date de dépôt: **28.04.2010**

(51) Int Cl.:
***A61L 27/24*** *(2006.01)*          ***A61L 27/58*** *(2006.01)*

(86) Numéro de dépôt international:
**PCT/EP2010/055681**

(87) Numéro de publication internationale:
**WO 2010/125086 (04.11.2010 Gazette 2010/44)**

(54) **NOUVEAUX MATÉRIAUX EN COLLAGÈNE ET PROCÉDÉS D'OBTENTION**

NEUE KOLLAGENMATERIALIEN UND GEWINNUNGSVERFAHREN DAFÜR

NOVEL COLLAGEN MATERIALS AND METHODS FOR OBTAINING SAME

(84) Etats contractants désignés:
**AT BE BG CH CY CZ DE DK EE ES FI FR GB GR
HR HU IE IS IT LI LT LU LV MC MK MT NL NO PL
PT RO SE SI SK SM TR**

(30) Priorité: **28.04.2009 FR 0952768**

(43) Date de publication de la demande:
**07.03.2012 Bulletin 2012/10**

(73) Titulaire: **BIOM'UP**
**69800 Saint Priest (FR)**

(72) Inventeurs:
• **GAGNIEU, Christian**
**F-69680 Chassieu (FR)**
• **FOREST, Patricia**
**F-69003 Lyon (FR)**
• **PICOT, Sylvain**
**F-69006 LYON (FR)**

(74) Mandataire: **Regimbeau**
**139, rue Vendôme**
**69477 Lyon Cedex 06 (FR)**

(56) Documents cités:
**FR-A- 2 724 563          US-A1- 2006 099 268
US-A1- 2009 054 984**

• **ROUSSEAU C F ET AL: "In vitro cytocompatibility
of porcine type I atelocollagen crosslinked by
oxidized glycogen" BIOMATERIALS, ELSEVIER
SCIENCE PUBLISHERS BV., BARKING, GB
LNKD- DOI:10.1016/S0142-9612(01)00276-9, vol.
23, no. 6, 15 mars 2002 (2002-03-15), pages
1503-1510, XP004348160 ISSN: 0142-9612**

**Description**

L'invention concerne de nouveaux matériaux en collagène et plus particulièrement des membranes, des tubes, des films, des éponges, des gels, des matrices et des fils de collagène. Ces matériaux combinent d'excellentes propriétés d'élasticité et de résistance mécanique. L'invention a également pour objet un procédé de préparation de matériaux en collagène à partir de collagène fibreux acide de tendons comportant également une réticulation contrôlée du collagène. L'invention se rapporte également à un collagène fibreux acide de tendons présentant des fibres longues, à son procédé d'obtention ainsi qu'à ses applications dans la fabrication notamment de membranes, de tubes, de films, d'éponges, de gels, de matrices et de fils de collagène.

Le collagène est une protéine connue depuis l'antiquité. Depuis de nombreuses années, il est utilisé pour la fabrication de dispositifs médicaux car ses propriétés physico-chimiques et biologiques sont remarquables. Historiquement employé pour la réalisation de compresses hémostatiques, sa biocompatibilité et son activité sur la cicatrisation font de lui un matériau de choix pour la confection de biomatériaux comme des membranes pour la Régénération Tissulaire Guidée en chirurgie dentaire et pour l'enduction de matériaux de renforts pariétaux et de prothèses vasculaires, pour faciliter leur intégration et garantir leur étanchéité.

Des matériaux entièrement en collagène sont disponibles sur le marché comme des compresses hémostatiques, des membranes pour la prévention des adhérences, des conduits de régénération nerveuse (WO 2007/147739, FR 2 810 889).

A condition que le procédé d'extraction du collagène conduise à une purification suffisante, ces produits sont parfaitement biocompatibles et remplissent bien leurs fonctions.

En fonction des indications et donc de la durée de résorption souhaitée dans l'organisme, le collagène peut être réticulé. Les procédés de réticulation du collagène sont bien connus et largement décrits. On citera par exemple EP 0 862 468 et US 4,931,546. Le document US 2006/099268 décrit un procédé de préparation d'un matériau en collagène à partir d'une solution aqueuse de collagène comportant une étape de coagulation et fibrillation par traitement à l'ammoniac, suivie après rinçage d'une étape de réticulation photochimique, puis d'une étape de séchage. L'utilisation du glycogène oxydé comme réticulant du collagène a également déjà été décrite dans FR 2 877 669 ainsi que dans les publications Forest *et al.* et Rousseau *et al.* En fonction de la nature et de la proportion de réticulant, ainsi que des conditions de réticulation (pH, temps de réaction), il est relativement aisé de faire varier de façon contrôlée la durée de résorption d'un matériau. Toutefois, la difficulté de la réticulation réside notamment dans le choix du taux de réticulation, qui doit permettre de produire un matériau stable, reproductible, avec un temps de résorption voulu, présentant des propriétés mécaniques définies compatibles avec l'application. Ce problème est d'autant plus important pour des matériaux à longue durée de vie car plus un matériau collagénique est réticulé plus il est rigide. Cette caractéristique physique qui se traduit par une augmentation de la susceptibilité à la déchirure et une diminution de la résistance à la suture, peut être rédhibitoire pour certaines utilisations en chirurgie. Or, il n'existe pas à l'heure actuelle de procédé de réticulation permettant de contrôler de manière précise et reproductible le taux de réticulation du collagène, et donc de maitriser la rigidité du matériau final.

Ainsi, dans certains cas, et notamment lorsque les produits sont soumis à de fortes contraintes mécaniques par le chirurgien et/ou le patient après l'implantation, ces matériaux collagéniques existants atteignent leurs limites. C'est le cas par exemple du produit décrit dans le brevet FR 2 877 669 et dans la publication scientifique FOREST *et al* (2007). Ces matériaux remplissent parfaitement leurs fonctions mais dans des cas extrêmes où les sutures sont délicates ou les contraintes plus fortes, le matériau n'est pas suffisamment résistant.

Les propriétés mécaniques d'un produit en collagène dépendent de trois facteurs :

- le choix du niveau de structuration du collagène,
- le choix du réticulant et du taux de réticulation,
- les procédés de préparation et de mise en forme du matériau.

Face aux nouvelles techniques chirurgicales et aux champs des possible ouverts pour les chirurgiens, ces derniers recherchent de plus en plus de produits biodégradables suffisamment résistants pour être suturés et mis en tension, éventuellement sans pour autant qu'ils soient renforcés par un treillis textile par exemple. C'est notamment le cas des membranes pour la régénération tissulaire guidée. Pour la réalisation d'un tel matériau, l'homme du métier choisira un collagène très structuré et réticulera le collagène fortement. Il obtiendra cependant un produit très rigide, cassant et non manipulable aisément par le chirurgien. Le collagène choisi pourra être un collagène dit fibreux c'est-à-dire peu déstructuré mais l'absence de contrôle sur le procédé d'extraction limite les possibilités des matériaux produits avec ces collagènes. La réticulation sera réalisée soit par immersion soit par mise en contact de vapeur dé formaldéhyde ou de glutaraldéhyde par exemple sans permettre l'obtention d'une réticulation souple et contrôlée. Ainsi, il n'existe pas aujourd'hui de produits collagéniques à la fois résistants mécaniquement (tension, suture) tout en étant souples et conformable et présentant un temps de résorption adapté (plusieurs mois).

La fabrication de matériaux en collagène réticulé comprend habituellement la préparation d'une solution aqueuse de collagène, l'ajout éventuel d'un agent de réticulation, la mise en forme du matériau par coulage ou moulage de la solution de collagène, l'évaporation du solvant puis le traitement du matériau obtenu par des procèdes physiques ou chimiques, dans des bains, vapeurs ou sous pression réduite permettant la formation de liaisons de réticulation, l'élimination et/ou la désactivation de l'agent de réticulation résiduel ou toute molécule indésirable , puis une nouvelle étape de séchage du matériau si la forme finale du matériau le nécessite.

Ces procédés selon l'état de la technique nécessitent de nombreuses manipulations du matériau en collagène et ne permettent pas un contrôle satisfaisant de l'étape de réticulation en particulier au niveau de la densité des liaisons réticulantes et de la structuration du collagène. Ces procédés ne permettent notamment pas une étape de coagulation/fibrillation ou une étape de coagulation/fibrillation/réticulation suffisamment progressive pour permettre une organisation tridimensionelle des molécules de collagène. De ce fait, les matériaux réticulés généralement obtenus sont soit résistants et rigides soit souples mais peu résistants.

La présente invention décrit maintenant un procédé permettant l'obtention d'un matériau en collagène et notamment des membranes de collagène ayant une résistance élevée à la traction et à la déchirure, tout en conservant une souplesse et une élasticité suffisante pour les applications chirurgicales notamment.

En effet, les inventeurs ont développé un nouveau procédé de préparation de matériau en collagène comprenant un traitement de collagène fibreux à l'état humide avec de l'ammoniac à l'état gazeux lors de sa mise en forme. Ce traitement progressif à l'ammoniac gazeux permet d'obtenir à la fois une coagulation et une fibrillation du collagène fibreux lors de sa mise en forme. L'invention se rapporte donc également à un procédé de mise en forme de collagène fibreux et notamment à l'utilisation d'une base faible telle que l'ammoniac à l'état gazeux pour assurer la coagulation et fibrillation complète du collagène sous forme de gel. Ce procédé permet aussi la réticulation concomitante du collagène lors de sa mise en forme par l'ajout direct de l'agent de réticulation dans la solution de collagène fibreux avant la mise en forme de celui-ci par coulage ou moulage. Le taux de réticulation du collagène peut ainsi être contrôlé de manière précise et reproductible.

Par ailleurs, le procédé peut permettre une réticulation sensiblement homogène dans le matériau, c'est-à-dire que le taux de réticulation peut être sensiblement identique à l'extérieur et à l'intérieur du matériau. Ceci permet notamment à ce matériau d'avoir des propriétés améliorées par rapport aux matériaux dont la réticulation est beaucoup plus importante à l'extérieur qu'à l'intérieur, voire inexistante à l'intérieur.

Le procédé peut ainsi conduire à un matériau présentant des propriétés mécaniques et/ou de résorption améliorée. Sans vouloir être lié par cette théorie, il est possible que la vitesse de résorption soit mieux maitrisée grâce à l'homogénéité de réticulation et/ou de structure dans le volume du matériau. Par « mieux maitrisé », on peut entendre notamment au sens de la présente invention que d'un échantillon de produit à l'autre, en particulier issu de deux lots de fabrications différents, et/ou d'un patient à l'autre la vitesse de résorption présente des différences plus faible que dans le cas de produits issu de procédé connus de l'art antérieur.

Par ailleurs, le matériau peut présenter une résorption sensiblement constante, ou linéaire, en fonction du temps. Ceci peut ainsi conduire à un relargage progressif des parties dégradées.

En revanche, dans les cas des matériaux existants, dans lesquels la réticulation n'est pas homogène en volume, la dégradation peut être « brutale ». Sans vouloir être lié par cette théorie, il est probable qu'une fois que la dégradation de la partie externe est accomplie, la partie interne, présentant un degré de réticulation moindre, est dégradée beaucoup plus, voire très, rapidement. Ceci peut donc provoquer une augmentation rapide de la quantité de produits de dégradation, ce qui peut causer de réactions inflammatoires, voire des poussées inflammatoires brutales.

Préférentiellement, ces procédés sont mis en oeuvre avec du collagène fibreux et de préférence avec du collagène présentant des fibres longues. Les inventeurs ont également développé un procédé de préparation de collagène fibreux acide de tendons ayant la particularité de présenter des fibres particulièrement longues et élastiques. Le collagène fibreux obtenu est utile pour la préparation de matériaux de collagène notamment de membranes, films, éponges, gels, matrices, fils et tubes selon l'invention.

Les procédés selon la présente invention permettent de fabriquer des dispositifs médicaux et notamment des membranes, films, éponges, gels, matrices, fils et tubes présentant des propriétés en terme de résistance mécanique, élasticité, suturabilité et conformabilité jamais atteintes avec les procédés classiques de production de matériaux en collagène. Les matériaux obtenus par les procédés selon l'invention peuvent être utilisés en chirurgie générale et spécialisée, notamment pour les chirurgies urologique, gynécologique, cardiaque, thoracique, vasculaire, articulaire, digestive, plastique, spinale, neurologique, orthopédique, traumatologique, dentaire, maxillofaciale et stomatologique, pour la cicatrisation guidée ou la substitution des tissus (dure mère, gencive, os, nerfs, tendons, ligaments, viscères, péricarde, péritoine, tissus conjonctifs en général, derme, muscle, cartilage) quelle que soit la forme que peut prendre le matériau ainsi fabriqué.

Ces matériaux, compte tenu de leurs caractéristiques peuvent être utilisés sous forme de membrane ou film comme barrière de cicatrisation guidée et/ou anti-adhérentielle dans toute chirurgie ou la séparation de deux organes ou tissus est nécessaire pendant les phases de cicatrisation, comme guide de régénération sous forme de tube ou de manchons fabriqué par le chirurgien à sa convenance dans la régénération nerveuse et tendineuse, comme matrice de régénération pour l'ingénierie tissulaire lorsque le collagène est sous forme d'éponge par exemple. La conformabilité des produits due au collagène utilisé pour sa fabrication, les rend faciles à utiliser et leur permet d'épouser les formes des tissus sur lesquels ils sont posés tout en étant suturables afin d'être maintenus en place si besoin. En fonction du site d'implantation et de la durée de résorption souhaité, la réticulation peut être ajustée en faisant varier la proportion des groupements réactifs du réticulant et ceux du collagène dans la solution de collagène de départ (et/ou en augmentant ou en diminuant, la quantité d'ammoniac et/ou le temps de contact avec l'ammoniac). L'épaisseur du matériau peut également être ajustée aux mêmes fins.

Les applications préférées sont l'obtention de membranes (pour la régénération tissulaire guidée et substitution de tissus (dure-mère, péricarde,...) dans de nombreuses chirurgies), de tubes (pour la régénération nerveuse, par exemple pour la régénération tendineuse et ligamentaire), et de matrices (par exemple pour l'ingénierie tissulaire)

RESUME DE L'INVENTION

L'invention concerne un procédé de préparation d'un matériau en collagène comprenant les étapes suivantes :

a) préparation d'une solution aqueuse de collagène sous forme acide,
b) ajout d'un agent de réticulation aldéhydique non réactif à pH acide,
c) moulage ou coulage de la solution aqueuse de collagène,
d) coagulation et réticulation, de la solution aqueuse de collagène par traitement avec de l'ammoniac gazeux,
e) élimination de l'ammoniac en excès et obtention du matériau en collagène par séchage.

De préférence, la solution aqueuse de l'étape a) comprend 0,05 à 3% en poids de collagène sous forme acide.

Préférentiellement, le collagène sous forme acide est du collagène natif ou du collagène dénaturé.

Préférentiellement, la solution aqueuse de l'étape a) est préparée avec du collagène fibreux acide choisi parmi les collagènes de tendons de porc, de tendons de veau, de tendons d'agneau et de tendons de poulain.

Dans un mode de réalisation avantageux, la coagulation et optionnellement la réticulation de la solution de collagène s'effectuent par traitement avec de l'ammoniac gazeux pendant au moins 24 heures.

Dans un mode de réalisation avantageux, le procédé comprend l'ajout d'un agent de réticulation aldéhydique non réactif à pH acide, la coagulation et la réticulation de la solution aqueuse de collagène par traitement avec de l'ammoniac gazeux.

Avantageusement, l'agent de réticulation aldéhydique est choisi parmi le glycogène et les amylopectines aldéhydiques.

Plus avantageusement, l'agent de réticulation aldéhydique est du glycogène oxydé.

Préférentiellement, l'agent de réticulation aldéhydiques est ajouté dans des proportions allant de 0,05 à 5 pour le rapport CHO de l'agent de réticulation aldéhydique sur $NH_2$ du collagène.

Dans un mode de réalisation préféré du procédé selon l'invention, le matériau en collagène est une membrane, à l'étape c) la solution aqueuse de collagène est déposée sur un moule plan et à l'étape e) on élimine l'ammoniac en excès et l'on obtient une membrane en collagène par séchage.

L'invention se rapporte aussi à un matériau en collagène susceptible d'être obtenu par un procédé selon l'invention et/ou tel que décrit dans la présente description, plus particulièrement ledit matériau est obtenu par ledit procédé, voire ledit matériau est directement obtenu par ledit procédé.

Selon un autre de ses aspects, l'invention a également pour objet un matériau, notamment une membrane, en collagène présentant une réticulation homogène.

Par « réticulation homogène », on entend au sens de la présente invention que la différence entre la réticulation sur la surface externe et la réticulation à l'intérieur, notamment vers le, voire au, milieu du matériau est inférieure ou égale à 25 %, notamment inférieure ou égale à 20 %, en particulier inférieure ou égale à 15 %, voire inférieure ou égale à 10 %, et tout particulièrement inférieur ou égal à 5 %.

La différence de réticulation en % peut correspondre à la valeur absolue de [((réticulation externe - réticulation interne) / (réticulation interne + réticulation externe)) x 100].

La réticulation peut être évaluée par le nombre de moles de lysine libre par mg de matériau, notamment de la manière décrite dans l'exemple 7.

Le matériau, notamment la membrane, présentant une réticulation homogène peut avoir une épaisseur sèche d'au moins 50 $\mu$m, et tout particulièrement sa longueur, sa largeur et sa hauteur sont chacune supérieure ou égale à 50 $\mu$m.

Tout particulièrement le matériau se présente sous forme d'une membrane ayant une épaisseur sèche d'au moins 50 $\mu$m. La longueur et la largeur de ladite membrane pouvant être supérieure ou égale à 1 cm, voire à 5 cm.

Selon encore un autre de ses aspects, l'invention a pour objet un matériau en collagène présentant une température de dénaturation supérieure ou égalé à 3°C, notamment supérieure ou égale à 5°C, voire supérieure ou égale à 7°C, par rapport au matériau en collagène non-réticulé. Cette différence de température de réticulation peut être mesurée par Calorimétrie Différentielle à Balayage (DSC), notamment de la manière décrite dans l'exemple 8.

Le matériau en collagène réticulé peut présenter une augmentation de la température de dénaturation par rapport au matériau non réticulé d'au moins 5 %, en particulier 8%, notamment d'au moins 10 %.

Ce % d'augmentation de la température correspond à l'équation suivante :

$$[((\text{température de dénaturation du matériau réticulé} / \text{température du matériau non réticulé}) - 1) \times 100].$$

L'augmentation de la température de dénaturation du matériau réticulé par rapport à la température de dénaturation au matériau non réticulé peut permettre de vérifier que ledit matériau est effectivement réticulé.

De préférence, ce matériau en collagène peut consister en une membrane en collagène, un film en collagène, un fil en collagène, un tube en collagène, une éponge en collagène ou un gel de collagène.

Dans un mode de réalisation préféré, l'invention a pour objet une membrane en collagène susceptible d'être obtenue par le procédé selon l'invention et ayant une épaisseur sèche comprise entre 30 et 200 $\mu$m, préférentiellement de 80 à 120 $\mu$m.

De préférence, la membrane en collagène est constituée d'une monocouche de collagène, non poreuse, d'épaisseur sèche comprise entre 50 et 150$\mu$m, préférentiellement de 80 à 120 $\mu$m.

La membrane de collagène obtenue selon l'invention a avantageusement une densité comprise entre 12 mg/cm$^2$ et 16 mg/cm$^2$, un taux de gonflement inférieur à 6, une résistance à la suture supérieure à 1N, une contrainte à la rupture supérieure à 4 MPa et un pourcentage de dégradation enzymatique à la trypsine inférieur à 35%.

L'invention a aussi pour objet une membrane en collagène ayant une épaisseur sèche comprise entre 80 et 120 $\mu$m, une densité comprise entre 12 mg/cm$^2$ et 16 mg/cm$^2$, un taux de gonflement inférieur à 6, une résistance à la suture supérieure à 1N, une contrainte à la rupture supérieure à 4 MPa et un pourcentage de dégradation enzymatique à la trypsine inférieur à 35%.

Plus préférentiellement, l'invention se rapporte à une membrane en collagène présentant un taux de gonflement compris entre 4 et 6, une résistance à la suture comprise entre 1N et 2.5 N, une contrainte à la rupture comprise entre 4 et 7 MPa et un pourcentage de dégradation enzymatique à la trypsine compris entre 20 et 35%.

Avantageusement, la membrane en collagène obtenue est armée d'un textile résorbable ou non.

DESCRIPTION DETAILLEE DE L'INVENTION

L'extraction du collagène à partir de tendons d'animaux jeunes conduisant à un collagène dont la longueur et l'élasticité des fibres permet ensuite lors de son utilisation dans la fabrication de dispositifs médicaux l'obtention de produits résistants mécaniquement, élastiques, suturables et conformables, est fait par un procédé de préparation de collagène fibreux acide de tendons comprenantes étapes suivantes:

a) gonflement des tendons de porc, dé veau, d'agneau, de poulain ou de leurs mélanges dans une solution aqueuse d'acide acétique entre 0.1 et 0.5 M pendant au moins sept jours,
b) broyage mécanique des tendons pour obtenir une suspension aqueuse,
c) précipitation et lavages du collagène fibreux à partir de la suspension aqueuse de l'étape b),
d) déshydratation du collagène,

De préférence, l'extraction de collagène fibreux s'effectue à partir de tendons d'animaux jeunes ayant moins de 10 mois et plus préférentiellement à partir de tendons de porcs ayant moins de 10 mois.

La première étape comporte alors le prélèvement des tendons de pieds de porcs de moins de 10 mois (les tendons peuvent également être prélevés sur des veaux, agneaux et poulains), le nettoyage, l'élimination au maximum des tissus conjonctifs et autres tissus non tendineux puis le découpage des tendons en morceaux d'1 cm de long environ et le rinçage à l'eau.

Le gonflement s'effectue pendant au moins 7 jours et jusqu'à 15 jours, préférentiellement 15 jours dans un bain d'acide acétique à une concentration comprise entre 0,1 et 0,5M préférentiellement 0,3M sous agitation à raison de 1kg de tendons dans un volume compris entre 20 et 30 L préférentiellement 25L.

La deuxième étape consiste en un broyage doux, permettant la libération de fibres tendineuses longues à partir des fragments gonflés de tendons. Le broyage d'un volume du bain de gonflement contenant les morceaux de tendons gonflés s'effectue par exemple pendant 2 min à 3000tr/min puis des étapes comprenant chacune une dilution du milieu par de l'eau suivie d'un broyage dans les mêmes conditions sont réalisées jusqu'à l'obtention d'une pâte de concentration en matière sèche comprise entre 4,8 et 6,5 g/ kg.

La troisième étape consiste en la précipitation du collagène fibreux à partir de la pâte issue du broyage, et à sa purification selon des procédés classiques. Cette étape peut comporter une ou plusieurs précipitations par du chlorure de sodium à une concentration finale comprise entre 0,45M et 1,2 M et plus particulièrement à la concentration de 0,6M et une ou plusieurs étapes de lavage du collagène précipité dans une solution de NaCl 0,45-1,2 M, préférentiellement 0,6M. En général, il est également prévu une étape d'inactivation virale dans une solution d'hydroxyde de sodium 1N, à 20°C pendant 1h. De par son action hydrolytique sur les protéines non collagéniques, cette étape constitue une purification supplémentaire. A l'issue de cette étape, de nouveaux lavages avec du NaCl 0,6M sont réalisés. Afin de déshydrater le collagène et d'éliminer les sels, on effectue ensuite un traitement par l'acétone qui conduit à l'obtention d'une fibre sèche.

Ce procédé particulier appliqué à des tendons conduit à un collagène différent des collagènes existants car composé de longues fibres sans pour autant contenir des morceaux de tissus et tout en conservant une part de collagène soluble.

Le protocole de mesure des fractions du collagène fibreux inférieures à 5$\mu$m et supérieures à 50 $\mu$m est le suivant :

- Préparation d'une solution aqueuse de collagène à 0,1 % sous agitation magnétique ou mécanique pendant 16 à 24h, (mise enjeu de 500mg).
- Dépôt de la solution sur une toile de nylon à mailles calibrées de 5$\mu$m ou 50$\mu$m montée sur un support circulaire de 9 cm de diamètre. La diffusion des molécules dans la toile a lieu à pression atmosphérique. La pression exercée sur la toile est considérée comme négligeable, la hauteur de la colonne d'eau ne saurait dépasser 4 cm pour une section de 63 cm$^2$.
- La solution sur la toile est agitée avec une pale carrée plate qui ne frotte pas sur la toile mais positionnée à quelques millimètre (au plus 5) au dessus de la toile. La vitesse d'agitation est de 80 tours / min.
- La largeur de la pale est 7 cm. Elle est positionnée au centre du support circulaire.
- Après arrêt de l'écoulement de la solution de collagène à travers la toile, le retentât est lavé par 50mL d'acide acétique 0,05M en respectant les différentiels de pression et jusqu'à arrêt de l'écoulement. Cette opération est renouvelée 3 fois.
- Les fractions sont ensuite récupérées (filtrat et retentât) et le collagène est précipité a partir de chacune des fractions par ajout de NaCl afin d'atteindre une concentration de 0,6M finale.

Le précipité est ensuite récolté par centrifugation ou filtration puis déshydraté par de l'acétone, séché sous pression réduite et pesé.

L'invention a pour objet un procédé de préparation d'un matériau en collagène caractérisé en ce qu'il comprend les étapes suivantes :

a) préparation d'une solution aqueuse de collagène sous forme acide,
b) ajout d'un agent de réticulation aldéhydique non réactif à pH acide,
c) moulage ou coulage de la solution aqueuse de collagène,
d) coagulation et réticulation de la solution aqueuse de collagène par traitement à l'ammoniac gazeux,
e) élimination de l'ammoniac et obtention du matériau en collagène.

La première étape des procédés selon l'invention consiste en la préparation d'une solution aqueuse de collagène. Par solution aqueuse de collagène on entend également une suspension de collagène.

Le procédé selon l'invention met en oeuvre du collagène sous forme acide, par collagène sous forme acide, on entend un collagène dont la majorité des fonctions carboxylique sont protonnées, et qui donne un pH acide en solution ou suspension dans l'eau.

De préférence, le procédé de préparation de matériau en collagène selon l'invention met en oeuvre du collagène fibreux acide.

Par collagène fibreux, on entend un collagène dans lequel les molécules de collagène ne sont pas ou très peu individualisées, qui est donc composé de fibres et fibrilles constituées de molécules de collagène naturellement liées entre elles par des liaisons faibles et covalentes, et par des agrégats de ces structures. Le collagène fibreux, notamment, est constitué de particules de grandes tailles (majoritairement supérieures à 5$\mu$m lorsqu'elles sont hydratées) qui donnent une suspension homogène par dispersion en milieu aqueux.

Le collagène fibreux peut être notamment un collagène fibreux de peau ou un collagène fibreux de tendons. Le collagène fibreux de peau comporte des fibres relativement courtes en raison de l'organisation naturelle du tissus, du collagène acido-soluble et des agrégats de petite taille. Le collagène de tendons comporte des fibres longues et très peu de collagène soluble.

De préférence, les procédés selon la présente invention sont mis en oeuvre avec du collagène fibreux de tendon, de préférence avec du collagène fibreux de tendons de porcs et plus préférentiellement avec du collagène de tendons de porcs jeunes de moins de 10 mois.

De manière avantageuse, les procédés de la présente invention utilisent du collagène fibreux acide de tendons préparé selon le procédé décrit ci-dessus et présentant des fibres longues.

La première étape consiste donc en la mise en solution du collagène dans de l'eau. Elle s'effectue selon des méthodes classiques décrites dans la littérature. Lorsque le collagène est un collagène fibreux acide, cette étape permet la mise en suspension de fibres entourées de collagène micro-fibrillaires et de collagène dit soluble ayant gardé une structure nécessaire à la fibrillation.

Typiquement, la solution aqueuse de collagène comprend entre 0.05% et 3 % en poids de collagène et de préférence entre 0.05, 0.1, 0.8 %, 1, 1.5, 2, 2.5 et 3% de collagène. Avantageusement la solution aqueuse comprend 0.8% de collagène en poids. Cette mise en solution s'effectue habituellement dans de l'eau par agitation mécanique de préférence sous pression réduite. La suspension ou solution peut également être chauffée à une température comprise entre 30°C et 100°C pendant 2 à 20 minutes pour dénaturer partiellement ou complètement le collagène.

Les procédés selon l'invention permettent d'obtenir divers matériaux en collagène en fonction de la mise en forme choisie lors du moulage ou du coulage. Le matériau en collagène peut ainsi notamment prendre la forme d'une membrane, d'une matrice, d'un film, d'un fil, d'un gel, d'un tube ou d'une éponge.

Le coulage ou le moulage d'une solution aqueuse de collagène sont bien connus de l'homme du métier et décrits dans la littérature. La deuxième étape est donc le coulage ou moulage de la solution de collagène dans des moules, l'épaisseur variant en fonction du matériau souhaité et en fonction de la surface du moule.

Les membranes de collagène sont des matériaux en deux dimensions résultant du séchage dans un moule plan d'une suspension homogène ou d'une solution de collagène contenant une proportion de fibres et de fibrilles. Le collagène peut être réticulé ou non. La concentration de la suspension séchée conditionne l'épaisseur du matériau final. Elle peut aller de quelques microns à plusieurs centaines de microns.

Un film de collagène est un matériau en deux dimensions résultats du séchage dans un moule plan d'une solution homogène de collagène. Le collagène peut être réticulé ou non. La concentration de la solution séchée conditionne l'épaisseur du matériau final. Les films et les membranes peuvent être repliés pour former des manchons qui peuvent être fermés si besoin par sutures ou collage. L'épaisseur peut varier de quelques microns à plusieurs centaines de microns. Un tube de collagène est un objet cylindrique en trois dimensions creux dont les parois peuvent être un film ou une membrane de collagène. Les tubes peuvent être obtenus par moulage autour d'un moule ou par extrusion. Le collagène peut être réticulé ou non. L'épaisseur des parois est conditionnée par la quantité de collagène déposé sur les moules ou mises enjeu dans la solution d'extrusion.

Un fil de collagène est un ensemble plein de collagène et dont la résistance mécanique est suffisante pour entrer dans la composition d'un fil multi brin plus gros, d'un textile composite ou non, d'un autre matériau de collagène.

Une éponge de collagène peut être obtenu par lyophilisation d'une solution ou d'une suspension de collagène (ou d'un mélange des deux). Avant ou après la lyophilisation, le collagène peut être réticulé. La lyophilisation conduit à des matériaux le plus souvent en 3 dimensions ou à des poudres.

Pour l'obtention d'une membrane ou d'un film, la solution de collagène peut être déposée sur un moule plan pour obtenir un matériau en deux dimensions après séchage de la solution ou de la suspension. Le film ou la membrane peuvent être obtenus par évaporation du solvant.

Des tubes en collagène sont obtenus par le dépôt de la solution ou de la suspension sur un moule cylindrique et séchage ou lyophilisation.

Pour l'obtention d'éponges, le retrait du solvant peut être réalisé par lyophilisation et non par évaporation du solvant sous forme liquide.

Il était déjà connu d'utiliser de l'ammoniaque pour la coagulation et mise en forme du collagène mais en général il s'agissait de l'utilisation de l'ammoniaque pour coaguler une solution ou un gel au cours de procédés d'extrusion par exemple. Le traitement avec l'ammoniaque était alors très rapide et en bains. Le procédé selon l'invention repose sur la vitesse de diffusion de l'ammoniac dans la solution de collagène, vitesse qui dépend essentiellement de la concentration de cette base à la surface de la solution. Le collagène et l'ammoniac sont laissés en contact un temps suffisant pour permettre la coagulation du collagène mais également sa fibrillation sur la totalité de la solution traitée. Ceci conduit à la préparation de matériaux de collagène présentant des propriétés mécaniques qui ne sont pas obtenus avec les procédés de l'état de la technique, au plan de la résistance à la traction, de l'élasticité et de la résistance à la suture.

La troisième étape est donc la coagulation du collagène par traitement avec de l'ammoniac pendant un temps suffisant pour permettre à la fois la coagulation et la fibrillation du collagène. Typiquement, le traitement avec l'ammoniac s'effectue pour une durée de 4, 8, 12, 24, 36, à 48 h. De préférence, le temps de traitement est supérieur à 24 ou 36 heures.

La quantité d'ammoniac est à ajuster pour permettre une augmentation de pH du gel de collagène d'un pH acide jusqu'à un pH au moins supérieur à 8. En effet, la réticulation du collagène débute lorsque le gel de collagène atteint un pH au moins supérieur à 8. Ce traitement long permet une augmentation progressive du pH du collagène conduisant non seulement à la coagulation mais également à la fibrillation de celui-ci. En fonction de la longueur des fibres de collagènes mises en oeuvre, cette fibrillation forme un maillage qui confère aux produits à la fois résistance mécanique et élasticité.

Dans un mode de réalisation préféré, l'ammoniac gazeux est préparé à partir d'une solution d'ammoniaque de laquelle il se dégage. On obtient généralement une quantité appropriée d'ammoniac gazeux avec une solution d'ammoniaque à au moins 30%, à une température comprise entre 10°C et 25°C. De préférence, cette étape est réalisée dans une enceinte fermée hermétiquement de manière que l'ammoniac gazeux se répande à l'intérieur de l'enceinte et entre en contact avec la solution de collagène, laquelle n'est pas en contact avec la solution d'ammoniaque.

Le gel de collagène obtenu est traité pour éliminer l'ammoniac en excès et il est soit conservé en l'état soit déshydraté. Pour cela le gel peut être placé dans une enceinte munie d'un système d'élimination de l'humidité et/ou d'un absorbeur d'ammoniac. Après élimination de l'ammoniac en excès, les membranes, films et tubes sont obtenus par déshydratation du gel sous courant d'air sec, tandis que les éponges, les matrices 3D ou les tubes également sont obtenues par lyophilisation du gel. Les gels peuvent être maintenus hydratés.

Dans ce procédé de préparation de matériaux en collagène, le processus de fibrillation a lieu dans un milieu liquide hautement visqueux. Cette fibrillation se produit de l'extérieur vers l'intérieur de la solution et progresse en profondeur au fur et à mesure de l'augmentation de pH liée à la diffusion de l'ammoniac. Elle se produit lorsque le pH a atteint une valeur supérieure à 4-5. L'avantage du procédé en vapeur d'ammoniac est que le produit n'a pas besoin d'être immergé dans des solutions de neutralisation, ce qui permet un gain de temps, de rentabilité et d'homogénéité.

Lorsque l'on souhaite augmenter la durée de résorption d'un dispositif médical collagénique et également renforcer ses propriétés mécaniques, le matériau de collagène doit être réticulé. Il existe de nombreuses méthodes de réticulation du collagène bien connues de l'homme du métier. Elles sont classées en deux grandes catégories: les réticulations physiques comme par exemple la déshydratation thermique et les réticulations chimiques par ajout ou mises en présence d'agents réticulants. Les réticulants du collagène les plus connus sont les agents aldéhydiques en particulier le formaldéhyde et le glutaraldehyde. Ces procédés de réticulation peuvent bien sur être utilisés sur les matériaux de collagènes obtenus ci-dessus.

Ainsi, afin d'augmenter la résistance mécanique du collagène ou des matériaux de collagène on peut donc procéder à leur réticulation. Elle s'effectue par exemple par immersion du matériau de collagène dans un bain comprenant un agent réticulant choisi parmi le formaldéhyde, le glutaraldéhyde, le glycogène oxydé et l'amylopectine oxydée.

De façon particulièrement avantageuse, la réticulation peut au contraire s'opérer en une seule étape mais de façon séquentielle avec la coagulation et la fibrillation du collagène. Dans ce cas, on introduit dans la solution de collagène de départ un agent réticulant aldéhydique qui ne réagit pas avec le collagène à pH acide puis on procède au traitement avec l'ammoniac pour l'obtention d'un pH au moins supérieur à 8.

L'agent de réticulation aldéhydique est de préférence choisi parmi les polysaccharides et plus particulièrement les polysaccharides oxydés. De préférence, l'agent de réticulation aldéhydique est choisi parmi le glycogène oxydé et les amylopectines oxydées. Des agents de réticulation utilisables dans les procédés selon la présente invention sont par exemple l'amidon oxydé, le dextrane oxydé, la cellulose oxydée connus de l'homme du métier. Préférentiellement, l'agent de réticulation aldéhydique est du glycogène oxydé.

L'agent de réticulation est ajouté dans des proportions allant de 0.05, 0.1, 0.5, 1, 1.5, 2, 2.5, 3, 3.5, 4, 4.5 à 5 pour le rapport CHO de l'agent de réticulation aldéhydique sur $NH_2$ du collagène. Les proportions d'agent de réticulation pourront être ajustées par l'homme du métier en fonction du taux de réticulation désirée. La quantité d'agent de réticulation à introduire dans la solution de collagène pourra ainsi être déterminée en utilisant les connaissances générales de l'homme du métier.

De préférence, on procède alors à la préparation d'une solution aqueuse concentrée (15%) du polysaccharide oxydé choisi. Le taux d'oxydation et la quantité de réticulant à ajouter sont à apprécier en fonction de la résorption souhaitée et des propriétés mécaniques recherchées. Il est alors possible d'ajouter le réticulant au collagène en quantité parfaitement contrôlé et reproductible (à la différence des réticulations par vapeur de formol par exemple ou par immersion dans des bains). Ici, seul le réticulant introduit peut réagir. La solution de réticulant est ajoutée à la solution de collagène avant coulage ou mise en forme, c'est-à-dire à la fin de l'homogénéisation sous pression réduite. Le milieu résultant est un mélange homogène du collagène et du réticulant mais les liaisons entre les deux ne se créent pas tant que l'ensemble n'a pas atteint un pH basique. Les étapes suivantes sont identiques à celles de la fibrillation du collagène, la fibrillation et la réticulation se faisant successivement et dans cet ordre.

L'homme du métier saura adapter la quantité d'ammoniac et la durée d'exposition pour atteindre la fibrillation et la réticulation souhaitée.

Cette étape du procédé selon l'invention est remarquable pour plusieurs raisons. La réticulation par les polysaccharides aldéhydiques a déjà été décrite dans la littérature (Gagnieu CH et Forest PO, EP 0862 468). Cette réticulation peut se faire soit par immersion des produits à réticuler dans une solution du polysaccharide oxydé soit par introduction dans le produit du polysaccharide oxydé puis immersion du produit sec dans un bain permettant la réaction de réticulation (augmentation du pH). En général, le changement de pH est effectué par un tampon et compte tenu du principe de la réticulation bien connu (réaction de Maillard réaction des CHO du réticulant sur les NH2 du collagènes), on évite de réaliser le changement de pH par des bases présentant elle-même des résidus amines. Ainsi, en présence d'ammoniac, la théorie prédit que le polysaccharide oxydé va réagir avec l'amine de l'ammoniac et par conséquent s'inactiver. La réticulation ne peut donc pas avoir lieu.

En pratique, il s'avère que la présence d'ammoniac modifie bien le pH du gel de collagène pour permettre la fibrillation mais également la réticulation. De façon tout à fait surprenante, la réticulation a lieu à un taux efficace car la réaction de Maillard qui aurait due se produire entre l'ammoniac et les aldéhydes du réticulant, inactivant ce dernier est soit absente, soit de faible amplitude, soit non compétitive avec la réaction de réticulation des groupements aldéhyde du polysaccharide oxydé sur les aminés des lysines du collagène. Ceci est prouvé par le fait que les matériaux réticulé par ce biais ne sont plus solubles en milieu aqueux acide, présentent des dégradations au contact d'enzymes protéolytiques inférieures à des matériaux non réticulés et que les propriétés mécaniques des matériaux sous forme hydratée, et notamment la résistance mécanique, sont également améliorées par rapport à un matériau non réticulé.

L'invention a également pour objet un matériau de collagène susceptible d'être obtenu par les procédés selon l'invention. De préférence, le matériau en collagène est réticulé. Ce matériau en collagène peut par exemple consister en une membrane de collagène, un fil de collagène, un tube de collagène, une éponge de collagène ou un gel de collagène.

L'invention se rapporte ainsi également à des films, fils et tubes de collagène susceptibles d'être obtenus par les procédés selon la présente invention. Dans un mode de réalisation avantageux, l'invention se rapporte à des membranes de collagène susceptibles d'être obtenues par les procédés selon l'invention.

Les procédés selon l'invention permettent la préparation de membranes sèches d'épaisseur variables pouvant aller de quelques microns à quelques centaines de microns. L'épaisseur généralement utilisée pour assurer la cicatrisation guidée (=conservation des plans de clivage) ou la substitution de tissus en chirurgies urologique, gynécologique, cardiaque, thoracique, vasculaire, articulaire, digestive, plastique, spinale, neurologique, orthopédique, traumatologique, dentaire, maxillofaciale et stomatologique, pour la cicatrisation guidée des tissus (dure mère, gencive, os, nerfs, tendons, ligaments, viscères, péricarde, péritoine, tissus conjonctifs en général, derme, muscle, cartilage) est comprise entre 30 et 200 $\mu$m.

L'invention à donc pour objet des membranes de collagène susceptibles d'être obtenues par les procédés selon l'invention ayant une épaisseur sèche comprise entre 30 et 200$\mu$m. De préférence, ces membranes sont réticulées.

Avantageusement, ces membranes sont constituées d'une monocouche de collagène, non poreuse, d'épaisseur sèche comprise entre 50 et 150$\mu$m.

Dans un mode de réalisation, l'invention a pour objet des membranes de collagène ayant une épaisseur sèche comprise entre 80 et 120 $\mu$m, une densité comprise entre 12 mg/cm$^2$ et 16 mg/cm$^2$, un taux de gonflement inférieur à 6, une résistance à la suture supérieure à 1N, une contrainte à la rupture supérieure à 4 MPa et un pourcentage de dégradation enzymatique à la trypsine inférieur à 35%.

Dans un mode de réalisation préféré, l'invention a pour objet les membrane de collagène préalablement décrites présentant un taux de gonflement compris entre 4 et 6, une résistance à la suture comprise entre 1 et 2.5 N, une contrainte à la rupture comprise entre 4 et 7 MPa et un pourcentage de dégradation enzymatique à la trypsine compris entre 20 et 35%.

Le taux de gonflement est mesuré comme suit : 20mg de produit sont immergés dans du Phosphate Buffer Saline IX pH 7,4 pendant 60min à 37°C. Au terme de l'heure, l'excès d'eau est retiré avec du papier absorbant et l'échantillon est à nouveau pesé. Le taux de gonflement est calculé par le rapport de la masse du produit humide / masse du produit sec.

Les mesures de contraintes mécaniques (résistance à la suture et contrainte) sont mesurées sur une éprouvette humidifiée de 5m de large à l'aide d'un banc d'essai de traction. En ce qui concerne la suture, un fil de suture de type tresse polyamide 3/0 est passé à travers la membrane puis la force maximale à appliquer pour rompre la suture est mesurée à l'aide d'un banc d'essai de traction.

Pour déterminer la dégradation enzymatique à la trypsine, des fragments de produit de masses comprises entre 10 et 20mg sont immergés dans 3mL de PBS 1X pH 7,6, 500 unités de trypsine sont ajoutées à l'échantillon. Après 48 heures de dégradation, les échantillons digérés sont récoltés, déshydratés et pesés. La perte de masse par rapport à la masse de départ est ensuite calculée.

L'invention concerne aussi des membranes en collagène armées d'un textile, résorbable ou non. Ces membranes années avec un textile constituent un renfort pariétal et sont particulièrement adaptées pour la chirurgie viscérale et uro-gynécologique ou un patch ligamentaire pour renforcer, prolonger ou remplacer un ligament ou un tendon.

L'invention a donc également pour objet un matériau composite comprenant, ou consistant en, un textile recouvert sur une face par un matériau en collagène tel que décrit ci-dessus.

Il peut notamment s'agir d'un textile portant une membrane de collagène selon l'invention sur l'une de ces faces. De telles tissus prothétiques et leurs procédés de fabrication sont par exemple décrits dans le US 6,451,032.

Des membranes en collagène armées d'un tissu selon l'invention peuvent par ailleurs être fabriquées selon des procédés bien connus de l'homme du métier.

Dans le cadre de la présente invention un tel procédé peut comprendre les étapes suivantes :

- préparation d'une solution aqueuse de collagène sous forme acide,
- ajout d'un agent de réticulation aldéhydique non réactif à pH acide,
- moulage ou coulage de la solution aqueuse de collagène,
- dépôt du textile sur le collagène,
- coagulation et réticulation de la solution aqueuse de collagène par traitement à l'ammoniac,
- élimination de l'ammoniac et obtention du matériau en collagène.

Les membranes armées d'un textile sur une face obtenues selon le procédé ci-dessus conviennent particulièrement pour la chirurgie pariétale.

Alternativement, le procédé peut comprendre les étapes suivantes :

- préparation d'une solution aqueuse de collagène sous forme acide,
- ajout d'un agent de réticulation aldéhydique non réactif à pH acide,
- moulage ou coulage de la solution aqueuse de collagène,
- inclusion du textile dans le collagène,
- coagulation et réticulation de la solution aqueuse de collagène par traitement à l'ammoniac,
- élimination de l'ammoniac et obtention du matériau en collagène.

Les textiles comprenant ainsi une membrane selon l'invention des 2 côtés sont particulièrement adaptés pour la chirurgie ligamentaire par exemple.

L'invention a donc encore pour objet un matériau composite comprenant, voire consistant en, un textile recouvert sur deux, en particulier sur chacune, de ses faces par un matériau en collagène telle que décrit ci-dessus, en particulier le textile peut être inclus dans le matériau de collagène.

D'autres procédés permettant d'associer un matériau en collagène selon l'intention avec un textile sont connus de l'homme du métier.

L'invention concerne donc également un matériau en collagène selon l'invention, et notamment une membrane, associé à un textile.

Les procédés selon l'invention conduisent aussi à la préparation de tubes pour assurer le guidage d'organes en chirurgie nerveuse, tendineuse et ligamentaire, vasculaire. Les membranes peuvent également pour cette indication être enroulées et fermées sous forme de manchon par suture et/ou collage.

Enfin, les procédés permettent la préparation de matrices 3D d'une épaisseur supérieure à $200\mu m$, poreuses ou non, permettant entre autre l'ensemencement de cellules préalablement ou non à l'implantation chirurgicale du matériau pour des applications en médecine régénérative ou permettant l'obtention de patchs suturables et élastiques pour dés applications cardiaques, régénération de dure mère, guidage de tissus mous et durs.

<u>EXEMPLES</u>

<u>Exemple 1 : Production de collagène fibreux acide de tendons</u>

→ Gonflement des tendons

1kgs de tendons de pieds de porcs sont nettoyés pour enlever les parties musculaires et aponévrotiques. Ils sont immergés dans 25L d'une solution aqueuse d'acide acétique 0,3M pendant 10 jours à 20°C (+/- 2°C) sous agitation lente.

→ Broyage des tendons

3L de la suspension obtenue sont broyés à 3000tr/min dans un broyeur à couteaux pendant 2 minutes. Le milieu est dilué par 2L d'eau et l'ensemble est homogénéisé pendant 1 minute. Le milieu est filtré sur filtre de porosité $200\mu m$ et le filtrat est ajusté à 0,6M de NaCl pour faire précipiter le collagène.

→ Récupération du collagène et lavages

La suspension est filtrée ou centrifugée pour séparer le précipité du surnageant. Le précipité est récolté et lavé dans 10L de NaCl 0,6M sous agitation pendant au moins 1heure ; le précipité est à nouveau récolté par filtration sur toile où centrifugation. L'étape de lavage peut être réalisée le nombre de fois désiré en fonction de la pureté du collagène final.souhaité (idéalement 2 fois).

→ Inactivation virale et lavages

Le collagène précipité et essoré est dissous à 1% dans de l'eau pendant 16 heures sous agitation. La concentration du milieu est amenée à 1M de NaOH et la solution est agitée pendant 1h à 20°C. A l'issue de l'étape d'inactivation, la solution est neutralisée par de l'acide chlorhydrique 6M jusqu'à précipitation du collagène. Le collagène est récupéré par filtration ou centrifugation. Le collagène peut à nouveau être lavé dans 10L de NaCl 0,6M puis récolté par filtration sur toile ou centrifugation. L'étape de lavage peut être réalisée le nombre de fois désiré en fonction de la pureté du collagène final souhaite (idéalement 2 fois).

→ Récolte et séchage

A l'issue du procédé de purification, le collagène précipité est essoré puis séché dans des bains d'acétone. Le collagène est finalement séché sous flux d'air contrôlé pour éliminer l'acétone résiduelle puis conservé par exemple à -20°C.

Exemple 2 : Caractérisation d'un lot de collagènes fibreux acide de tendons

603mg de collagène fibreux acide de tendons présentant un taux d'humidité de 17,05% sont mis en dispersion dans 500mL d'eau déminéralisée pendant 16h sous agitation magnétique. Une toile de porosité calibrés de $50\mu m$ est placé sur un support cylindrique de 9cm de diamètre au dessus d'un récipient. Un volume de solution de collagène est coulé sur la toile de façon à ne pas dépasser 4cm de hauteur. Une pale de 7c de diamètre est placée à 2mm de la toile et une agitation de 80 tours / min est effectuée, la solution de collagène s'écoule progressivement à travers la toile. Lorsque le volume contenu dans la chambre supérieure ne diminue plus, le système est rechargé de façon à ne jamais dépasser 4cm de hauteur .Ces opérations sont réalisées jusqu'à épuisement de la solution préparée. A équilibre du système, le retentât est lavé par 3 x 50mL d'acide acétique 0,05M avec le même système, en respectant les différentiels de pression. La fraction supérieure est collectée.
La fraction inférieure est récupérée et l'analyse est poursuivie de la même façon sur une toile calibrée de $5\mu m$ de porosité. Le retentât ainsi que le filtrat sont collectés. Les 3 fractions, c'est-à-dire le retentât de la filtration sur $50\mu m$, le retentât et le filtrat de la filtration sur $5\mu m$, sont amenées à 0,6M de NaCl et le collagène est récupéré par centrifugation puis séché par 2 bains d'acétone 70 et 3 bains d'acétone 100. L'excès d'acétone est éliminé par séchage sous flux d'air. Les fractions sont pesées et rapportés à la masse totale collectées. L'analyse conduit à 6,5% de fibres retenues sur un filtre de $50\mu m$, 27% de fibres ayant traversé un filtre de $5\mu m$ et 66,5% comprises entre 5 et $50\mu m$.

Exemple 3 : Préparation d'un film / membrane de collagène réticulé #1

800mg de collagène fibreux, acide de tendons sont mis en suspension sous agitation mécanique dans 100mL d'eau pendant 16 heures. La suspension visqueuse est coulée dans un moule à raison de 4mg de collagène/cm². Le moule contenant la solution de collagène est placée dans une enceinte fermée hermétiquement de 3L contenant 2 mL d'ammoniaque 30% pendant 24 heures à 20°C. Le gel est placé dans une enceinte permettant d'éliminer l'excès d'ammoniac avec un absorbeur d'ammoniac et d'humidité pour obtenir un film d'une épaisseur voisine de $40\mu m$. Le film peut être utilisé tel quel ou réticulé par immersion dans un bain de formaldéhyde, glutaraldehyde, glycogène oxydé, amylopectine oxydé de diverses concentrations pendant des temps pouvant varier entre 2 minutes et 24h. Les réticulants sont inactivés par immersion du film dans une solution de glycine 0,1M ; pH 8 pendant 2 heures. Le film est ensuite à nouveau séché.
Par exemple, le film obtenu après le premier séchage est plongé pendant 1 h dans un bain de formaldéhyde 0,1 % pH 8 puis rincé dans un bain de glycine 0,1 M pH 8 pendant 2 heures. Après un rinçage à l'eau, le film est à nouveau séché.

Exemple 4 : Préparation d'une membrane de collagène réticulée #2

Pour l'obtention d'une membrane contenant 10mg de collagène/ cm², 100g de collagène sont mis en suspension dans 12,5L d'eau sous agitation mécanique pendant 16 heures. Parallèlement, 2,5g de glycogène oxydé dissout à 15% dans du tampon phosphate pH 7,7 sont préparés et ajoutés à la suspension au terme des 16 heures. Après homogénéisation, la solution est coulée dans un moule d'1m² (ou équivalent). Le ou les moulues contenant la solution de collagène est placé dans une enceinte fermée hermétiquement de 300L environ contenant 160mL d'ammoniaque 32% répartis de façon homogène pendant 48 heures à 20°C. A l'issue de la phase de fibrillation, réticulation, le ou les gels sont placés dans une enceinte permettant d'éliminer l'excès d'ammoniac avec un absorbeur d'ammoniac et d'humidité pour obtenir une membrane d'épaisseur voisine de 100μm.

Exemple 5 : Mesure du taux de gonflement d'une membrane préparé à l'exemple 4

3 échantillons de 20,5 / 22 et 20mg de produit sont pesés précisément et immergés dans 3ml de PBS IX pH 7,4 pendant 1 heure à 37°C. Au terme de l'heure, l'excès d'eau de chaque échantillon est retiré et les échantillons sont à nouveau pesés. Les résultats sont les suivants :

|  | Masse prélevée (mg) | Masse après gonflement (mg) | Taux de gonflement |
|---|---|---|---|
| Echantillon 1 | 20,5 | 110 | 5,5 |
| Echantillon 2 | 22 | 127,6 | 5,8 |
| Echantillon 3 | 20 | 114 | 5,7 |
| Moyenne | | | 5,66 |

Exemple 6 : Éponge de collagène fibreux acide réticulé par les amylopectines oxydées

Une solution aqueuse de collagène fibreux acide est obtenue par mélange de 0,8g dans 100mL d'eau. Le milieu est agité pendant 16 heures à 20°C. 28mg d'amylopectine contenant 1,4 moles d'aldéhydes / moles de saccharides sont chauffés à 75°C dans 1ml de tampon phosphate 0,1M, pH 7,7 jusqu'à dissolution complète. Après refroidissement à 20°C, cette solution est versée sous agitation dans la solution de collagène à 0,8%. Le milieu homogène est coulé dans un moulé sur une hauteur de 5mm et transférée dans une enceinte fermée hermétiquement d'un volume d'environ 3L contenant 3ml d'ammoniaque à 28% pendant 16 heures. Le dispositif contenant le gel est ensuite placé dans une enceinte hermétiquement close contenant un absorbeur d'ammoniac jusqu'à disparition totale de l'ammoniac dans l'enceinte. Le gel de collagène est ensuite congelé puis lyophilisé pour donner une éponge d'atélocollagène réticulé.

Exemple 7 : Mesure de l'homogénéité de réticulation

Un gel d'environ 1 cm d'épaisseur a été préparé par la procédure décrite dans l'exemple 4 jusqu'à l'étape de réticulation.
A l'issue de cette étape de réticulation, le gel a été démoulé, coupé en deux, environ vers le milieu, dans le sens horizontal et les deux fragments sont sèches séparément. Un échantillon « externe » et un échantillon « interne » de matériau (environ 10mg chacun) sont prélevés respectivement sur ce qui correspond à la partie externe et à la partie interne du gel.
Le taux de réticulation est déterminé par un dosage des amines restées libres dans le collagène par du TNBS (acide 2,4,6 trinitrobenzène sulfonique). Ce réactif TNBS réagit spécifiquement sur les amines des résidus lysine et des acides aminés terminaux libre. Les échantillons interne et externe sont prélevés et incubés dans une solution eaux propanol (1ml) à 60°C pendant 1h. 500μL de bicarbonate 8% et 1ml de TNBS dilué au 1/120e sont ajoutés. La réaction a lieu pendant 3 heures à 40°C.
Après refroidissement, 200μL d'HCl 6N sont ajoutés pour stopper la réaction. L'excès de TNBS est extrait par 5mL d'acétate d'éthyle. Une hydrolyse acide (3mL d'HCl 6N pendant 1h15) libère tous les acides aminés. Les acides aminés terminaux N-TNBS sont extraits de la même manière que pour l'excès de TNBS.
Après une dilution adéquate, l'absorbance de la phase aqueuse est mesurée à 345nm. Le coefficient d'extinction molaire du complexe à 345nm, mesuré selon le protocole décrit par Kakade et *al.,* est de $1,46.10^{-4}$ $M^{-1}.cm^{-1}$, il permet de calculer la quantité de lysine restées libres dans la membrane. Le résultat est exprimé en μmol de lysine libre par mg de membrane.
Pour une membrane réticulée avec du glycogène oxydé à raison de 0,4 CHO du glycogène oxydé pour 1 $NH_2$, les résultats sont les suivants :

|  | μmol de lysine libres /mg dé membrane |
|---|---|
| Echantillon externe | 0,161 |
| Echantillon interne | 0,150 |

La différence de réticulation est donc de 3,5 %, soit ((0,16.1- 0,150)/(0,161 + 0,150) x 100). Les taux de réticulation de la partie externe et de la partie interne sont donc sensiblement équivalents. La réticulation est donc bien sensiblement homogène sur toute l'épaisseur du matériau.

Exemple 8 : Mesure de la réticulation

Une membrane dite « réticulée » a été préparée selon le procédé décrit en exemple 4, y compris l'étape d'addition de l'ammoniac.

Une membrane dite « non réticulée » a été préparée selon le procédé décrit en exemple 4 dans lequel l'étape d'addition de l'ammoniac est omise.

La réticulation peut être caractérisée par une mesure de DSC (Differential Scanning Calorimetry ou calorimétrie différentielle par balayage). Cette méthode mesurée les différences des échanges de chaleur entre un échantillon à analyser et une référence.

Elle permet de déterminer les transitions de phase :

- la température de transition vitreuse ($T_g$)
- les températures de fusion ou dénaturation
- les enthalpies de réaction (pour connaître les taux de réticulation des polymères).

Les analyses sont réalisées sous balayage d'un gaz inerte (par exemple, l'azote ou l'argon) pour éviter toute réaction du matériau à étudier avec l'atmosphère du four.

En ce qui concerne un collagène, la réticulation entraîne l'augmentation de la température de dénaturation. Pour prouver que l'incubation de la solution de collagène contenant le réticulant dans des vapeurs d'ammoniaque conduit à la formation de liaisons chimiques stables de réticulation entre le collagène et le réticulant. Le profil en DSC d'une membrane réticulée et une membrane non réticulée ont été effectuées.

|  | Température de dénaturation (°C) |
|---|---|
| membrane réticulée | 49,62 |
| Membrane non réticulée | 42,37 |

La température de dénaturation de la membrane réticulée est donc bien supérieure à celle de la membrane non réticulée. La réaction entre le réticulant aldéhydique et le collagène a donc bien lieu lors de l'incubation des solutions dans les vapeurs d'ammoniaque.

REFERENCES

Forest PO, Karoum R, Gagnieu CH. Influence of gradual introduction of hydrophobic groups (stearic acid) in denatured atelocollagen on fibroblasts behavior in vitro, J Biomed Mater Res A. 2007 Mar 1;80(3):758-67.

Gagnieu CH, Forest PO. In vivo biodegradability and biocompatibility of porcine type 1 atelocollagen newly crosslinked by oxidized glycogen, Biomed Mater Eng. 2007;17(1): 9-18).

Rousseau, C.F. and C.H. Gagnieu, In vitro cytocompatibility of porcine type I atelocollagen crosslinked by oxidized glycogen. Biomaterials, 2002. 23(6): p. 1503-10.

Kakade ML., Liener IE, Détermination of available lysine in proteins, Anal Biochem, 1969 ; 27(2) : 273-280

REFERENCES BREVET

WO 2007/147739
FR 2 810 889
FR 2 877 669
EP 0 862 468
US 4,931,546

**Revendications**

1. Procédé de préparation d'un matériau en collagène **caractérisé en ce qu'**il comprend les étapes suivantes :

    a) préparation d'une solution aqueuse de collagène natif ou dénaturé sous forme acide,
    b) ajout d'un agent de réticulation aldéhydique non réactif à pH acide,
    c) moulage ou coulage de la solution aqueuse de collagène,
    d) coagulation et réticulation de la solution aqueuse de collagène par traitement avec de l'ammoniac gazeux,
    e) élimination de l'ammoniac en excès et obtention du matériau en collagène par séchage.

2. Procédé de préparation d'un matériau en collagène selon la revendication 1 **caractérisé en ce que** la solution aqueuse de l'étape a) comprend 0,05 à 3% en poids de collagène sous forme acide.

3. Procédé de préparation d'un matériau en collagène selon la revendication 1 ou 2 **caractérisé en ce que** la solution aqueuse de l'étape a) est préparée avec du collagène fibreux acide choisi parmi les collagènes de tendons de porc, de tendons de veau, de tendons d'agneau et de tendons de poulain.

4. Procédé de préparation d'un matériau en collagène selon l'une quelconque des revendications 1 à 3, **caractérisé en ce que** lors de l'étape d) la coagulation et la réticulation de la solution de collagène s'effectuent par traitement avec de l'ammoniac gazeux pendant au moins 24 heures.

5. Procédé de préparation d'un matériau en collagène selon l'une quelconque des revendications 1 à 4, **caractérisé en ce que** l'agent de réticulation aldéhydique est choisi parmi le glycogène et les amylopectines aldéhydiques.

6. Procédé de préparation d'un matériau en collagène selon l'une quelconque des revendications 1 à 4, **caractérisé en ce que** l'agent de réticulation aldéhydique est du glycogène oxydé.

7. Procédé de préparation d'un matériau en collagène selon l'une quelconque des revendications 5 ou 6, **caractérisé en ce que** l'agent de réticulation aldéhydique est ajouté dans des proportions allant de 0,05 à 5 pour le rapport CHO de l'agent de réticulation aldéhydique sur $NH_2$ du collagène.

8. Procédé de préparation d'un matériau en collagène selon l'une quelconque des revendications 1 à 7, **caractérisé en ce que** le matériau en collagène est une membrane, **en ce que** à l'étape c) la solution aqueuse de collagène est déposée sur un moule plan et **en ce que** à l'étape e) on élimine l'ammoniac en excès et l'on obtient une membrane en collagène par séchage.

9. Matériau en collagène obtenu par le procédé selon l'une quelconque des revendications 1 à 8, présentant une réticulation, homogène, présentant une différence entre la réticulation sur la surface extérieure et la réticulation à l'intérieur du matériau inférieure ou égale à 25 %.

10. Matériau en collagène selon la revendication 9, présentant une température de dénaturation supérieure ou égale à 3°C par rapport au matériau en collagène non-réticulé.

11. Matériau en collagène réticulé selon l'une quelconque des revendications 9 ou 10, présentant une augmentation de la température de dénaturation par rapport au matériau non réticulé d'au moins 5 %.

12. Matériau en collagène selon l'une quelconque des revendications 9 à 11, consistant en une membrane de collagène, un film de collagène, un fil de collagène, un tube de collagène, une éponge de collagène ou un gel de collagène.

13. Matériau en collagène selon l'une quelconque des revendications 9 à 11, consistant en une membrane de collagène ayant une épaisseur sèche comprise entre 80 et 120 $\mu$m, une densité comprise entre 12 mg/cm$^2$ et 16 mg/cm$^2$, un taux de gonflement inférieur à 6, une résistance à la suture supérieure à IN, une contrainte à la rupture supérieure à 4 MPa et un pourcentage de dégradation enzymatique à la trypsine inférieur à 35%.

14. Matériau composite comprenant un textile recouvert sur une de ses faces ou sur chacune de ses faces par un matériau tel que décrit selon l'une quelconque des revendications 9 à 12, en particulier le textile peut être inclus dans le matériau de collagène.

**Patentansprüche**

1. Verfahren zur Gewinnung eines Kollagenmaterials, **dadurch gekennzeichnet, dass** es die folgenden Schritte umfasst:

   a) Herstellen einer wässrigen Lösung nativen oder denaturierten Kollagens in saurer Form,
   b) Hinzufügen eines nicht mit saurem pH reaktiven aldehydischen Vernetzungsmittels,
   c) Formen oder Gießen der wässrigen Kollagenlösung,
   d) Koagulieren und Vernetzen der wässrigen Kollagenlösung durch Behandlung mit gasförmigem Ammoniak,
   e) Entfernen des überschüssigen Ammoniaks und Gewinnung des Kollagenmaterials durch Trocknung.

2. Verfahren zur Gewinnung eines Kollagenmaterials nach Anspruch 1, **dadurch gekennzeichnet, dass** die wässrige Lösung von Schritt a) 0,05 bis 3 Gew.-% Kollagen in saurer Form umfasst.

3. Verfahren zur Gewinnung eines Kollagenmaterials nach Anspruch 1 oder 2, **dadurch gekennzeichnet, dass** die wässrige Lösung von Schritt a) mit saurem Faserkollagen hergestellt ist, das aus den Kollagenen von Schweinesehnen, von Kälbersehnen, von Lämmersehnen und von Fohlensehnen ausgewählt ist.

4. Verfahren zur Gewinnung eines Kollagenmaterials nach einem der Ansprüche 1 bis 3, **dadurch gekennzeichnet, dass** bei Schritt d) die Koagulation und die Vernetzung der Kollagenlösung durch Behandlung mit gasförmigem Ammoniak während mindestens 24 Stunden erfolgen.

5. Verfahren zur Gewinnung eines Kollagenmaterials nach einem der Ansprüche 1 bis 4, **dadurch gekennzeichnet, dass** das aldehydische Vernetzungsmittel aus dem Glykogen und den aldehydischen Amylopektinen ausgewählt ist.

6. Verfahren zur Gewinnung eines Kollagenmaterials nach einem der Ansprüche 1 bis 4, **dadurch gekennzeichnet, dass** das aldehydische Vernetzungsmittel oxidiertes Glykogen ist.

7. Verfahren zur Gewinnung eines Kollagenmaterials nach einem der Ansprüche 5 oder 6, **dadurch gekennzeichnet, dass** das aldehydische Vernetzungsmittel in Anteilen von 0,05 bis 5 für das Verhältnis CHO des aldehydischen Vernetzungsmittels zum $NH_2$ des Kollagens hinzugefügt wird.

8. Verfahren zur Gewinnung eines Kollagenmaterials nach einem der Ansprüche 1 bis 7, **dadurch gekennzeichnet, dass** das Kollagenmaterial eine Membran ist, dass in Schritt c) die wässrige Kollagenlösung auf eine ebene Form aufgebracht wird und dass in Schritt e) das überschüssige Ammoniak entfernt wird und dass eine Kollagenmembran durch Trocknung gewonnen wird.

9. Durch das Verfahren nach einem der Ansprüche 1 bis 8 gewonnenes Kollagenmaterial, das eine homogene Vernetzung aufweist, einen Unterschied zwischen der Vernetzung auf der äußeren Fläche und der Vernetzung im Innern des Materials unter oder gleich 25 % aufweisend.

10. Kollagenmaterial nach Anspruch 9, das eine Denaturierungstemperatur von über oder gleich 3 °C im Verhältnis zum nicht vernetzten Kollagenmaterial aufweist.

11. Vernetztes Kollagenmaterial nach einem der Ansprüche 9 oder 10, das eine Erhöhung der Denaturierungstemperatur im Verhältnis zum nicht vernetzten Material von mindestens 5 % aufweist.

12. Kollagenmaterial nach einem der Ansprüche 9 bis 11, das aus einer Kollagenmembran, einem Kollagenfilm, einem Kollagenfaden, einem Kollagenschlauch, einem Kollagenschwamm oder einem Kollagengel besteht.

13. Kollagenmaterial nach einem der Ansprüche 9 bis 11, das aus einer Kollagenmembran mit einer trockenen Dicke zwischen 80 und 120 um inklusive, einer Dichte zwischen 12 mg/cm$^2$ und 16 mg/cm$^2$ inklusive, einer Schwellenrate unter 6, einer Nahtfestigkeit über 1 N, einer Zerreißspannung über 4 MPa und einem Prozentsatz enzymatischer Zersetzung gegenüber Trypsin unter 35 % besteht.

14. Verbundmaterial, das ein Textil umfasst, das auf einer seiner Flächen oder auf jeder seiner Flächen mit einem Material nach einem der Ansprüche 9 bis 12 bedeckt ist, wobei das Textil insbesondere in das Kollagenmaterial eingeschlossen sein kann.

**Claims**

1. A method for preparing a collagen material **characterized in that** it comprises the following steps:

   a) preparing an aqueous solution of native or denatured collagen in acid form,
   b) adding an aldehyde cross-linking agent that is non-reactive at acidic pH,
   c) molding or casting the aqueous collagen solution,
   d) coagulating and cross-linking the aqueous collagen solution by treating with ammonia gas,
   e) removing excess ammonia and obtaining the collagen material by drying.

2. The method for preparing a collagen material according to claim 1, **characterized in that** the aqueous solution of step a) comprises 0.05% to 3% by weight of collagen in acid form.

3. The method for preparing a collagen material according to claim 1 or 2, **characterized in that** the aqueous solution of step a) is prepared with acidic fibrous collagen selected from collagens of pig tendons, calf tendons, lamb tendons and foal tendons.

4. The method for preparing a collagen material according to any one of claims 1 to 3, **characterized in that** during step d) coagulation and cross-linking of the collagen solution are carried out by treating with ammonia gas for at least 24 hours.

5. The method for preparing a collagen material according to any one of claims 1 to 4, **characterized in that** the aldehyde cross-linking agent is selected from glycogen and aldehyde amylopectins.

6. The method for preparing a collagen material according to any one of claims 1 to 4, **characterized in that** the aldehyde cross-linking agent is oxidized glycogen.

7. The method for preparing a collagen material according to any one of claims 5 or 6, **characterized in that** the aldehyde cross-linking agent is added in proportions ranging from 0.05 to 5 for the CHO ratio of the aldehyde cross-linking agent on the $NH_2$ of the collagen.

8. The method for preparing a collagen material according to any one of claims 1 to 7, **characterized in that** the collagen material is a membrane, **in that** in step c) the aqueous collagen solution is deposited on a flat mold and **in that** in step e) excess ammonia is removed and a collagen membrane is obtained by drying.

9. Collagen material obtained by the method according to any one of claims 1 to 8, with homogeneous cross-linking, with a difference between cross-linking on the outside surface and cross-linking inside the material of less than or equal to 25%.

10. Collagen material according to claim 9, with an increase in denaturation temperature greater than or equal to 3°C in relation to non-cross-linked collagen material.

11. Cross-linked collagen material according to any one of claims 9 or 10, with an increase in denaturation temperature in relation to non-cross-linked material of at least 5%.

12. Collagen material according to any one of claims 9 to 11, consisting of a collagen membrane, a collagen film, a collagen thread, a collagen tube, a collagen sponge or a collagen gel.

13. Collagen material according to any one of claims 9 to 11, consisting of a collagen membrane with a dry thickness between 80 μm and 120 μm, a density between 12 mg/cm$^2$ and 16 mg/cm$^2$, a swelling ratio less than 6, a suture retention strength greater than 1 N, a yield strength greater than 4 MPa and a percentage of enzymatic degradation by trypsin less than 35%.

14. Composite material comprising a textile covered on one of its sides or each of its sides by a material as described according to any one of claims 9 to 12, in particular the textile may be included in the collagen material.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 2007147739 A
- FR 2810889
- EP 0862468 A
- US 4931546 A
- US 2006099268 A
- FR 2877669
- US 6451032 B

**Littérature non-brevet citée dans la description**

- **FOREST PO ; KAROUM R ; GAGNIEU CH.** Influence of gradual introduction of hydrophobic groups (stearic acid) in denatured atelocollagen on fibroblasts behavior in vitro. *J Biomed Mater Res A,* 01 Mars 2007, vol. 80 (3), 758-67
- **GAGNIEU CH ; FOREST PO.** In vivo biodegradability and biocompatibility of porcine type 1 atelocollagen newly crosslinked by oxidized glycogen. *Biomed Mater Eng,* 2007, vol. 17 (1), 9-18
- **ROUSSEAU, C.F. ; C.H. GAGNIEU.** In vitro cytocompatibility of porcine type I atelocollagen crosslinked by oxidized glycogen. *Biomaterials,* 2002, vol. 23 (6), 1503-10
- **KAKADE ML. ; LIENER IE.** Détermination of available lysine in proteins. *Anal Biochem,* 1969, vol. 27 (2), 273-280